# FASCICULE DE BREVET EUROPEEN

(11) **EP 2 215 053 B1**
(45) Date de publication et mention de la délivrance du brevet: **26.11.2014**
(21) Numéro de dépôt: 08847161.0
(22) Date de dépôt: 23.10.2008
(51) Int. Cl.: C07C 263/10, C07C 263/20, C07C 265/14

(54) **PROCEDE DE PREPARATION D'UN COMPOSE DE TYPE ISOCYANATE**
VERFAHREN ZUR HERSTELLUNG EINER ISOCYANATVERBINDUNG
METHOD FOR PREPARING AN ISOCYANATE COMPOUND

(30) Priorité: 09.11.2007 FR 0707870
(43) Date de publication de la demande: 11.08.2010
(73) Titulaire: Vencorex France, 69800 Saint-Priest (FR)
(72) Inventeur: LIMONGI, Eric, F-69130 Ecully (FR); INDEY, Sophie, F-38640 Claix (FR); COQUERET, Pierre, F-69340 Francheville (FR)
(74) Mandataire: Colombet, Alain André
(86) Numéro de dépôt international: PCT/EP2008/064372
(87) Numéro de publication internationale: WO 2009/059903

(56) Documents cités:
- WO-A-2004/056760
- FR-A- 1 476 755

## Description

La présente invention a pour objet un procédé de préparation d'un composé de type isocyanate.

L'invention concerne plus particulièrement la préparation d'un composé de type diisocyanate à partir du composé diaminé correspondant.

L'invention vise préférentiellement la préparation du toluènediisocyanate, de l'hexaméthylènediisocyanate et de l'isophoronediisocyanate.

Il est décrit selon FR 1 476 755 et son certificat d'addition 91 273, un procédé de préparation d'un composé de type isocyanate et plus particulièrement de diisocyanate comprenant une étape de réaction par mise en présence d'une amine, d'un excès de phosgène et d'un solvant, sous pression et une étape de distillation réactive sous pression.

Toutefois, l'efficacité de ce procédé, en termes de productivité d'appareillage, de taux opératoire (réduction de capacité et/ou arrêts pour maintenance et nettoyage), d'efficacité énergétique et de rendement peut être améliorée.

WO 2004/056760 décrit un procédé de préparation d'isocyanates (cyclo)aliphatiques par réaction dans un solvant organique de diamines (cyclo)aliphatiques primaires avec du phasgène dans la phase liquide.

La présente invention propose un procédé amélioré, présentant notamment une fiabilité et une efficacité de fonctionnement (taux opératoire) améliorées, par exemple en termes d'instabilités hydrodynamiques supprimées, de pulsations de pressions supprimées ou réduites dans les dispositifs, notamment dans le bouilleur de la colonne à distiller.

Ainsi, le procédé de l'invention permet d'obtenir le composé de type isocyanate, de préférence le toluènediisocyanate avec une meilleure productivité de la colonne de distillation réactive.

Par ailleurs, l'invention fournit un procédé de meilleure efficacité énergétique et qui permet une flexibilité plus grande pour optimiser le rendement de la réaction.

Il a maintenant été trouvé et c'est ce qui constitue l'objet de la présente invention, un procédé de préparation d'un composé de type isocyanate à partir de l'amine correspondante et de phosgène comprenant une étape de réaction sous pression, de l'amine et du phosgène, en présence d'un solvant organique suivie par une étape de distillation réactive sous pression qui est caractérisé par le fait qu'il comprend à la sortie de l'opération de phosgénation les étapes suivantes :
- une étape de séparation du mélange réactionnel. *(F₁)* issu de l'étape de phosgénation en un flux gazeux d'acide chlorhydrique et de phosgène *(F₃)* et en un flux liquide *(F₂)* comprenant essentiellement les précurseurs d'isocyanate dans le solvant organique,
- une introduction du flux gazeux séparé *(F₃)* dans la colonne de distillation, de préférence à mi-hauteur,
- une étape de préchauffage du flux liquide *(F₂)* à une température comprise entre 100°C et 250°C,
- une introduction du flux préchauffé *(F₄)* dans la colonne de distillation, en dessous de l'alimentation du flux *(F₃),*
- une opération de distillation du flux *(F₄)* conçue pour obtenir :
   ∘ en pied de distillation, *(F₁₀)*, l'isocyanate attendu dans le solvant organique et du phosgène résiduel,
   ∘ en tête de distillation, une phase gazeuse *(F₆)* comprenant du phosgène et l'acide chlorhydrique,
   ∘ dans une zone supérieure à l'alimentation du mélange réactionnel *(F₄)*, un flux liquide *(F₇)* comprenant très majoritairement du phosgène.

Une variante préférée du procédé de l'invention de préparation d'un composé de type isocyanate est caractérisée par le fait que l'étape de réaction de phosgénation de l'amine est conduite à une pression supérieure à la pression de distillation réactive et par le fait que le mélange réactionnel *(F₁),* à la sortie de l'étape de réaction, est soumis à une étape supplémentaire de détente de telle sorte que sa pression soit amenée au niveau de la pression de distillation.

Une autre variante préférée du procédé de l'invention consiste à ajouter une étape de déphosgénation du flux *(F₁₀)* successivement à l'étape de distillation.

Selon une caractéristique du procédé de l'invention, l'acide chlorhydrique produit au cours de la réaction de phosgénation et contenu dans *(F₃),* est éliminé dès la sortie de l'étape de phosgénation. Un avantage du procédé de l'invention provient du fait que l'élimination rapide du flux d'acide chlorhydrique permet d'éviter les réactions secondaires en particulier la formation de chlorhydrates d'amine.

De plus en éliminant l'acide chlorhydrique, on améliore le taux de transformation dans l'étape suivante de préchauffage du flux liquide avant l'introduction dans la colonne à distiller. Ainsi, on fait une décomposition quasi-totale des chlorures de carbamyle avant l'introduction du flux dans la colonne à distiller ce qui permet de réduire l'encrassement du bouilleur et de la colonne à distiller.

Par ailleurs, le fait de séparer le flux gazeux du flux liquide permet d'obtenir un gain sur l'appareillage de préchauffage qui peut être dimensionné plus petit.

Selon une autre caractéristique du procédé de l'invention, le flux liquide est préchauffé avant d'entrer dans la colonne à distiller. Le fait de préchauffer permet d'éviter des dégradations chimiques. En effet, la réaction de décomposition des chlorures de carbamyle étant une réaction endothermique, si le flux liquide entrant dans la colonne était froid, il faudrait encore plus chauffer en pied de colonne pour obtenir la réaction de décomposition des chlorures de carbamyle ce qui conduirait à davantage de dégradation chimique.

Dans le présent texte, on entend par « précurseurs d'isocyanate », les produits intermédiaires formés à savoir les chlorures de carbamyle correspondant à l'amine de départ.

Le procédé de l'invention convient pour la préparation de monoisocyanates et de polyisocyanates, de préférence de diisocyanates à partir de monoamines ou de polyamines, de préférence de diamines.

Ainsi, on donne ci-après, des schémas réactionnels du procédé de l'invention pour faciliter la compréhension de l'exposé de l'invention, sans pour autant lier la portée de l'invention à ceux-ci :
- pour une monoamine

   R - NH₂ + COCl₂ → R - NHCOCl + HCl (I)

   R - NHCOCl ⇆ R - NCO + HCl (II)

L'équation globale est la suivante :

R - NH₂ + COCl₂ → R - NCO + 2 HCl (III)

Le précurseur d'isocyanate, produit issu de la réaction (I), est appelé « chlorure de carbamyle ».
- pour une diamine

   H₂N - R - NH₂ + 2COCl₂ → ClOCHN - R - NHCOCl + 2 HCl (I')

   CIOCHN - R - NHCOCI ⇆ ClOCHN - R - NCO + HCl (II')

   ClOCHN - R - NCO ⇆ OCN - R - NCO + HCl (III')

L'équation globale est la suivante :

H₂N - R - NH₂ + 2COCl₂ → OCN - R - NCO + 4HCl (IV')

Les précurseurs de diisocyanate sont les produits issus des équations réactionnelles (I') et (II'). Ils sont appelés respectivement « dichlorure de carbamyle » et « monochlorure de carbamyle ».

Dans le présent texte, l'expression « distillation réactive » signifie non seulement la séparation de l'acide chlorhydrique et partiellement du phosgène d'un mélange comprenant l'isocyanate attendu selon une opération classique de distillation mais également une réaction de dissociation des chlorures de carbamyle intermédiaires en isocyanate.

Comme mentionné précédemment, le procédé de l'invention s'applique préférentiellement sur des substrats de type diamine bien que des monoamines ou des polyamines telles que des triamines peuvent être mises en oeuvre.

A titre d'exemple de monoamines convenant au procédé de l'invention, on peut citer notamment la n-butylamine, l'isopropylamine, l'aniline, la chloro-toluidine, la métatrifluorométhylaniline.

En ce qui concerne les substrats de départ de type diamine, il peut s'agir notamment d'une diamine aliphatique, cycloaliphatique et/ou aromatique.

Il est à noter que l'invention s'applique également aux mélanges de diamines, en particulier aux mélanges d'isomères obtenus au cours du procédé de synthèse des diamines.

Classiquement, les diamines aromatiques sont obtenues par nitration de l'hydrocarbure aromatique suivie par la réduction du groupe nitro.

Quant aux diamines aliphatiques et cycloaliphatiques, elles résultent notamment de la réduction des nitriles correspondants.

Les diamines répondent préférentiellement à la formule suivante :

H₂N - R - NH₂ (la)

dans ladite formule :
- R représente un groupe divalent choisi parmi les groupes alkylène, cycloalkylène ou arylène ou un enchaînement desdits groupes.

Dans la formule (la), on entend par « groupe alkylène », une chaîne hydrocarbonée linéaire ou ramifiée comprenant de 1 à 16 atomes de carbone, de préférence 6 atomes de carbone.

Comme exemples de tels groupes, on peut citer notamment, le groupe méthylène, hexaméthylène, méthylpentaméthylène ou 2,4,4-triméthylhexaméthylène.

Par « groupe cycloalkylène», on entend un carbocycle saturé ayant 5 ou 6 atomes de carbone dans le cycle, éventuellement substitué.

Le nombre de substituants varie généralement entre 1 et 4.

N'importe quel substituant peut être présent sur le cycle dans la mesure où il ne gêne pas la réaction. On peut mentionner particulièrement les groupes alkyle ou alkoxy ayant de 1 à 4 atomes de carbone, de préférence les groupes méthyle, éthyle ou méthoxy, les atomes d'halogène, fluor, chlore, brome, iode, de préférence fluor, les groupes perfluoroalkyle, de préférence le groupe trifluorométhyle.

Comme exemples préférés, on peut mentionner le groupe 1,4-cyclohexylène, méthylcyclohexylène.

Par « groupe arylène », on entend un groupe phénylène ou naphtylène éventuellement substitué.

On peut se référer à ce qui vient d'être décrit concernant le nombre et la nature des substituants présents sur le cycle.

Comme exemples de groupes arylène préférés, on peut citer le groupe phénylène, tolylène ou xylène et plus particulièrement les groupes aromatiques divalents suivants :

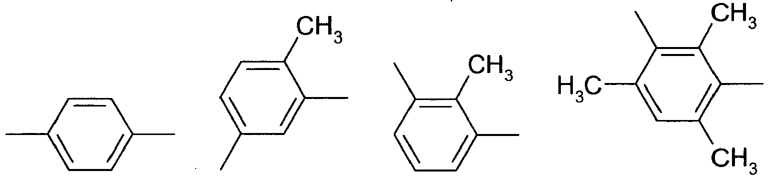

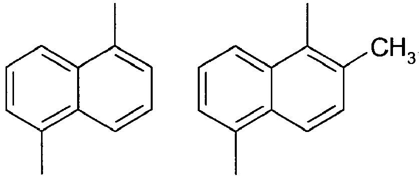

Comme mentionné dans la formule (la), R peut représenter un enchaînement de groupes alkylène et/ou cycloalkylène et/ou arylène.

On donne des exemples préférés de groupes divalents enchaînant des groupes alkylène et cycloalkylène :

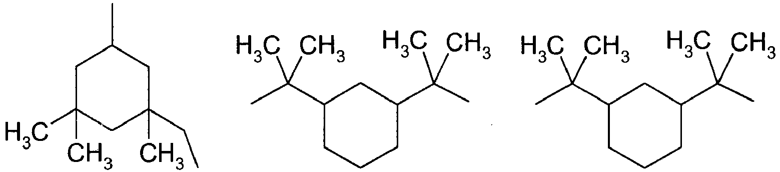

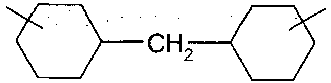

Comme exemples préférés d'enchaînements de groupes aromatiques, on peut citer spécifiquement les groupes biphényle éventuellement séparés par un groupe alkylène ou alkylidène ayant de 1 à 4 atomes de carbone ou par un hétéroatome, de préférence l'oxygène ou un groupe fonctionnel de préférence un groupe carbonyle : les groupes phényle pouvant porter des substituants.

A titre illustratif, on peut citer les groupes divalents suivants :

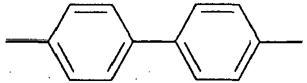

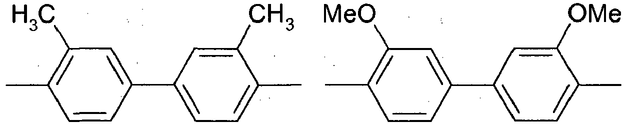

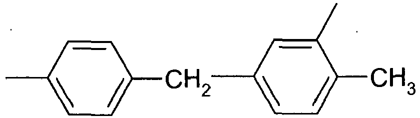

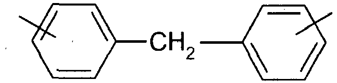

Dans la dernière formule, les valences sont en position 2,2', 2,4', 3,4', 4,4' de préférence en position 4,4'.

Parmi les diamines répondant à la formule (la), on choisit préférentiellement :
- l'hexaméthylènediamine,
- l'isophoronediamine,
- la 2,4-toluènediamine ou la 2,6-toluènediamine ou leur mélange, (TDA)
- la 4,4'- méthylènediphénylamine ou la 2,2'- méthylènediphénylamine ou la 2,4'- méthylènediphénylamine ou leur mélange (MDA),
- la 4,4'-méthylènedicyclohexyldiamine ou la 2,2'-méthylènedicyclohexyldiamine ou la 2,4'- méthylènedicyclohexyldiamine ou leur mélange (H₁₂MDA).

Parmi les diamines citées, le substrat de départ de choix est le mélange de 2,4-toluènediamine et de 2,6-toluènediamine. Généralement, il comprend 80 ± 1 % de 2,4-toluènediamine et 20 ± 1 % de 2,6-toluènediamine ainsi qu'à titre d'impuretés en une teneur généralement inférieure à 1 %, les autres isomères : 2,3-toluènediamine, 2,5-toluènediamine, 3,4-toluènediamine et 3,5-toluènediamine.

L'invention n'exclut pas le cas où l'amine est une triamine et l'on peut citer comme exemple, la 1,6,11-triaminoundécane.

### Phosgénation de l'amine.

Le procédé de l'invention consiste à effectuer la phosgénation de l'amine, en présence d'un solvant organique conduisant ainsi aux chlorures de carbamyle correspondants.

A cet effet, l'amine peut être introduite à l'état fondu ou bien dissoute dans un solvant organique.

Le choix du solvant doit satisfaire à plusieurs impératifs.

Il doit être inerte dans les conditions réactionnelles.

Il doit présenter un point d'ébullition supérieur à la température d'ébullition du phosgène mais inférieur à la température d'ébullition de l'isocyanate et impuretés formées.

Comme exemples préférés de solvants organiques, on fait appel aux hydrocarbures aliphatiques, cycloaliphatiques, aromatiques, halogénés ou non.

A titre d'exemples d'hydrocarbures aliphatiques ou cycloaliphatiques, on peut citer plus particulièrement les paraffines tels que, notamment, le cyclohexane, le méthylcyclohexane, la décaline ; les hydrocarbures aromatiques et plus particulièrement les hydrocarbures aromatiques comme notamment le benzène, le toluène, les xylènes.

En ce qui concerne les hydrocarbures halogénés aliphatiques ou aromatiques, on peut mentionner plus particulièrement, le monochlorobenzène, les dichlorobenzènes.

Comme autres types de solvants, on peut faire appel aux esters d'acides carboxyliques aliphatiques ou aromatiques. On peut mentionner notamment, les esters d'alkyle (C₁-C₈) d'acide butyrique, isobutyrique, hexanoïque, adipique, phtalique.

Parmi les solvants précités, on choisit avantageusement le toluène, le monochorobenzène ou les dichlorobenzènes.

L'amine présente généralement une concentration dans le solvant inférieure à 30 % en poids, de préférence entre 10 et 25 % en poids.

Selon le procédé de l'invention, on fait réagir l'amine avec le phosgène généralement préparé sur place par action entre le dichlore et le monoxyde de carbone. Il peut être également introduit sous forme de solution, dans le solvant organique choisi pour la réaction, à une concentration variant généralement entre 50 et 90 % en poids.

L'amine et le phosgène sont introduits de préférence en continu dans l'appareil de phosgénation où se déroule la réaction.

Le phosgène est généralement mis en oeuvre en grand excès.

Ainsi, le rapport entre le nombre de moles de phosgène et le nombre de fonctions amino varie le plus souvent entre 3 et 6, de préférence entre 3,5 et 5,5.

Dans le cas préféré de la mise en oeuvre d'une diamine, le rapport entre le nombre de moles de phosgène et le nombre de moles de diamine varie le plus souvent entre 6 et 12, de préférence entre 7 et 11.

La température de la réaction de phosgénation est comprise entre 80°C et 250°C, de préférence entre 110°C et 220°C et plus préférentiellement entre 110°C et 180°C.

Ce dernier intervalle est particulièrement préféré dans le cas de la préparation du toluènediisocyanate (TDI).

La réaction est conduite à une pression supérieure à la pression atmosphérique, de préférence à une pression comprise entre 5 et 80 bars, de préférence entre 15 et 55 bars et plus préférentiellement entre 40 et 50 bars lors de la préparation du TDI, la pression étant mesurée en sortie de réacteur.

En fin de réaction de phosgénation, on obtient un mélange réactionnel comprenant essentiellement les chlorures de carbamyle correspondant à l'amine de départ, le solvant organique, le phosgène en excès et l'acide chlorhydrique formé et des impuretés en faibles quantités tels que notamment des chlorhydrates d'amine.

### Pré-traitement du mélange réactionnel.

Conformément au procédé de l'invention, le mélange réactionnel *(F₁)* issu de l'étape de réaction de phosgénation est séparé en un flux gazeux *(F₃)* et un flux liquide *(F₂).*

Le mélange réactionnel *(F₁)* est en effet dirigé vers une enceinte de séparation qui assure la séparation des deux flux. Il peut s'agir d'un appareil cylindrique ne comprenant aucun interne, c'est-à-dire ni plateaux, ni garnissage.

On récupère en tête d'enceinte, un flux gazeux *(F₃)* comprenant de l'acide chlorhydrique et du phosgène et, en pied d'enceinte, un flux liquide *(F₂)* comprenant essentiellement du phosgène, les chlorures de carbamyle, le solvant organique, de l'acide chlorhydrique résiduel et des impuretés.

Conformément à une caractéristique du procédé de l'invention, la phase liquide est préchauffée à une température comprise entre 100°C et 250°C, de préférence entre 120°C et 220°C et préférentiellement entre 130°C et 160°C.

Cette dernière zone de température est préférée dans le cas de la préparation du TDI.

Le préchauffage permet, dans le cas de la préparation d'un monoisocyanate, de commencer la décomposition du chlorure de carbamyle en monoisocyanate correspondant.

Le préchauffage permet, dans le cas de la préparation d'un diisocyanate, d'une part de parfaire la décomposition du dichlorure de carbamyle en monochlorure de carbamyle et, d'autre part, de commencer la dissociation du monochlorure de carbamyle de la diamine en diisocyanate correspondant.

D'un point de vue pratique, le flux *(F₂)* est chauffé par passage dans un échangeur thermique qui peut être, notamment, un échangeur tubes calandre chauffé par condensation de vapeur d'eau ou par un fluide caloporteur approprié. Comme fluides caloporteurs convenant à l'invention, on peut mentionner notamment le solvant organique de la réaction, les esters lourds d'acides carboxyliques (par exemple, le phtalate d'octyle), les éthers aromatiques comme l'oxyde de biphényle et/ou l'oxyde de benzyle, le diphényle, les terphényles, les autres polyphényles éventuellement partiellement hydrogénés, les huiles paraffiniques et/ou naphténiques, résidus de distillation du pétrole, etc...

Parmi les fluides précités, le solvant organique du procédé est choisi préférentiellement.

Le flux *(F₂)* passe de l'enceinte de séparation à l'échangeur thermique par écoulement gravitaire ou par circulation forcée par exemple à l'aide d'une pompe, le plus souvent une pompe centrifuge.

On obtient un flux *(F₄)* enrichi en isocyanate qui est envoyé à la distillation, généralement à mi-hauteur d'une colonne de distillation.

Selon une variante de réalisation de l'invention, un autre mode de chauffage du flux *(F₂)* consiste à établir une boucle de recirculation sur l'enceinte de séparation et la chauffer par un échangeur thermique, notamment un échangeur tubes calandre. Plus précisément, le flux recirculé sort en pied d'enceinte, traverse de bas en haut un échangeur thermique et, en sortie d'échangeur, est introduit en tête de l'enceinte de séparation.

Selon le procédé de l'invention, on introduit également le flux gazeux *(F₃)* dans la colonne de distillation, dans une zone au-dessus de l'alimentation de *(F₄)* mais située au-dessus ou en dessous de la zone de récupération du flux liquide *(F₇)* tel que précisé ci-après.

Un mode de réalisation préférée de l'invention consiste à effectuer la réaction de phosgénation à une pression supérieure à la pression de distillation réactive.

Les pressions sont contrôlées par les vannes de régulation.

Comme mentionné précédemment, la réaction de phosgénation peut être conduite à une pression variant entre 5 et 80 bars, de préférence entre 15 et 55 bars et plus préférentiellement entre 40 et 50 bars

L'opération de distillation est conduite sous une pression comprise entre 3 et 30 bars, de préférence ente 10 et 30 bars.

Dans le cas où la pression de réaction est supérieure à la pression de distillation, il y a lieu de soumettre le mélange réactionnel à une détente afin de l'amener à la pression de distillation. Ainsi, la pression après détente est comprise entre 3 et 30 bars, de préférence ente 10 et 30 bars.

La détente est assurée à l'aide d'une vanne de détente (ou tout autre moyen) placée sur le conduit véhiculant le mélange réactionnel *(F₁)* de la zone de réaction à l'enceinte de séparation.

Ainsi, les dimensions de l'enceinte de séparation dépendent de la différence de pression entre la pression de la réaction et la pression de la distillation réactive.

L'Homme du métier est à même de dimensionner ladite enceinte sachant que les dimensions de l'enceinte seront d'autant plus grandes que la différence de pression est plus grande.

Le flux *(F₂)* au pied de l'enceinte est réchauffé comme décrit ci-dessus avant l'étape de distillation réactive.

On obtient un flux *(F₄)* enrichi en isocyanate qui est envoyé à la distillation, généralement à mi-hauteur d'une colonne de distillation.

### Distillation du mélange réactionnel.

L'étape suivante est une opération de distillation qui assure la séparation de l'acide chlorhydrique, et partiellement du phosgène du mélange réactionnel ainsi que la décomposition des chlorures de carbamyle.

Plus précisément, elle est conçue pour obtenir :
- en pied de distillation, *(F₁₀)*, l'isocyanate attendu dans le solvant organique et du phosgène résiduel,
- en tête de distillation, une phase gazeuse *(F₆)* comprenant du phosgène et l'acide chlorhydrique,
- dans une zone supérieure à l'alimentation du mélange réactionnel *(F₄)*, un flux liquide *(F₇)* comprenant très majoritairement du phosgène.

Le flux de phosgène *(F₇)*, peut être recyclé au niveau de l'étape de phosgénation.

L'étape de distillation est conduite sous pression comprise entre 3 et 30 bars, de préférence entre 10 et 30 bars, la pression étant mesurée en tête de colonne.

Cette étape vise à obtenir en pied l'isocyanate en solution dans le solvant organique dépourvu de chlorures de carbamyle.

A partir de ces informations, l'homme du métier est parfaitement en mesure de choisir les moyens à mettre en oeuvre en fonction du mélange de départ. On rappellera simplement ce qui suit. La taille (notamment le diamètre) des colonnes de distillation dépend des flux circulants et de la pression interne. Leur dimensionnement se fera donc principalement suivant le débit de mélange à traiter à l'alimentation et le taux de reflux. Le paramètre interne qu'est le nombre d'étages théoriques est déterminé notamment par la composition du mélange entrant *(F₄)* et la pureté des produits devant être obtenus en tête et en pied de distillation. On précisera que les colonnes sont garnies de plateaux et éventuellement de garnissage, comme cela est parfaitement connu de l'homme du métier. L'installation étant déterminée, l'homme du métier ajuste les paramètres de fonctionnement des colonnes.

Ainsi, le nombre d'étages théoriques, le taux de reflux et la température du flux d'alimentation *(F₄)* sont ajustés pour obtenir la dissociation des chlorures de carbamyle en isocyanate et le niveau de séparation souhaité pour l'acide chlorhydrique.

Pour effectuer la distillation, l'apport des calories en pied de colonne peut être fait notamment par un échangeur tubes calandre chauffé à la vapeur ou par un fluide caloporteur, par l'intermédiaire de serpentins de chauffage alimentés à la vapeur ou par un fluide caloporteur ou par tout autre dispositif équivalent.

Un mode de réalisation préféré consiste à chauffer le mélange en pied de distillation dans un échangeur thermique par prélèvement d'un flux *(F₅)* en pied qui circule en boucle. Plus précisément, le flux *(F₅)* sort en pied de distillation et traverse de bas en haut, un échangeur thermique et en sortie d'échangeur est introduit latéralement dans la partie inférieure de la colonne de distillation.

Un autre mode de réalisation consiste à effectuer une circulation forcée du flux (F₅) dans l'échangeur à l'aide d'une pompe.

Un autre mode d'exécution de l'invention, consiste à remplacer la colonne de distillation équipée de moyens de chauffage par un ensemble comprenant un réacteur équipé de moyens d'agitation et de chauffage surmonté d'une colonne de distillation.

Le flux gazeux *(F₆)*, en tête de colonne, comprenant majoritairement du phosgène et de l'acide chlorhydrique, est condensé de manière à récupérer un flux d'HCl gazeux *(F₈)* et un flux liquide *(F₉)* comprenant essentiellement du phosgène et minoritairement de l'acide chlorhydrique, ce dernier flux étant introduit, latéralement en tête de colonne, pour assurer le reflux dans la colonne.

La récupération du phosgène à partir du flux *(F₆)* se fait par condensation.

Le flux gazeux *(F₆)* est refroidi à une température comprise entre 30°C et 60°C, par passage dans un ou plusieurs condenseurs de préférence deux.

Le nombre de condenseurs est choisi en fonction des capacités réfrigérantes des liquides de refroidissement circulant dans les condenseurs à savoir l'eau, la saumure ou un fluide organique réfrigéré.

Dans le cas de condenseurs en série, la phase gazeuse en sortie du premier condenseur est introduite dans le deuxième condenseur.

On récupère en tête du ou des condenseurs, l'acide chlorhydrique gazeux *(F₈)* et en pied le phosgène comprenant de l'acide chlorhydrique résiduel *(F₉).*

Ce dernier flux est introduit, latéralement en tête de colonne de distillation.

Le produit *(F₁₀)* obtenu en pied de colonne de distillation comprend l'isocyanate attendu dans le solvant organique, du phosgène résiduel et des impuretés.

Il ne contient plus de chlorures de carbamyle. Si, exceptionnellement, on peut constater leur présence, leur teneur exprimée dans le flux *(F₁₀)* est généralement inférieure à environ 0,2 % en poids.

### Déphosgénation du flux de composé de type isocyanate.

Selon une variante préférée de l'invention, on élimine le phosgène présent dans le flux *(F₁₀)* par distillation dans une colonne à distiller.

La température de distillation est fonction de la nature de l'isocyanate, de la nature du solvant et de la pression de distillation.

Dans le cas de la distillation du TDI, la température de distillation, mesurée en pied de colonne varie entre 150°C et 200°C.

On récupère en pied de colonne, le flux *(F₁₅)* d'isocyanate dans le solvant organique et en tête de colonne le flux de phosgène et d'une fraction de solvant (entre 10 et 30 % en poids) *(F₁₂)* qui est ensuite condensé pour d'une part alimenter le reflux de la colonne *(F₁₄)* et d'autre part être recyclé *(F₁₃)* à l'étape de phosgénation.

Le flux gazeux *(F₁₂)* est refroidi à une température comprise entre 30°C et 60°C, par passage dans un ou plusieurs condenseurs de préférence deux.

Dans le cas de condenseurs en série, la phase gazeuse en sortie du premier condenseur est introduite dans le deuxième condenseur.

Les liquides condensés en sortie de condenseurs comprenant essentiellement du phosgène peuvent être réunis en un flux dont une partie *(F₁₄)* alimente le reflux de la colonne (7) et l'autre partie *(F₁₃)* peut être recyclée à l'étape de la phosgénation.

L'isocyanate peut être récupéré à partir de sa solution dans le solvant organique selon les procédés classiques de séparation, de préférence la distillation.

La présente invention a aussi pour objet une installation permettant la mise en oeuvre du procédé décrit ci-dessus, comprenant :
- un réacteur équipé de dispositifs d'introduction des réactifs (injecteurs),
- une enceinte de séparation assurant la détente éventuelle et la séparation du mélange réactionnel et des moyens de chauffage permettant d'augmenter la température du mélange réactionnel introduit dans la colonne de distillation,
- une première colonne de distillation équipée en pied de moyens de chauffage conçue pour séparer l'acide chlorhydrique et partiellement le phosgène du mélange réactionnel et pour dissocier les chlorures de carbamyle en isocyanate à partir dudit mélange,
- un ou deux condenseurs situés à la sortie du flux gazeux récupéré en tête de colonne de distillation conçus pour assurer le reflux dans la colonne de distillation.

Les différents appareillages sont reliés par des tuyauteries assurant la circulation et le recyclage des réactifs.

Conformément au mode de réalisation préféré de l'invention, cette installation comprend en outre entre le réacteur et l'enceinte de séparation, des moyens de détente, de préférence une vanne de réglage permettant d'amener la pression de la réaction à une pression voisine de la pression de la distillation.

Conformément à un mode de réalisation encore plus préféré de l'invention, cette installation comprend en plus, une unité de déphosgénation comprenant:
- une deuxième colonne de distillation dont l'entrée est alimentée par le pied de la première colonne, cette colonne étant conçue pour obtenir en pied, l'isocyanate attendu dans le solvant organique et les impuretés de réaction dans le solvant organique et en tête de colonne, le phosgène et une fraction de solvant,
- un ou deux condenseurs situés à la sortie du flux gazeux récupéré en tête de deuxième colonne de distillation conçus pour assurer le reflux dans la colonne de distillation.

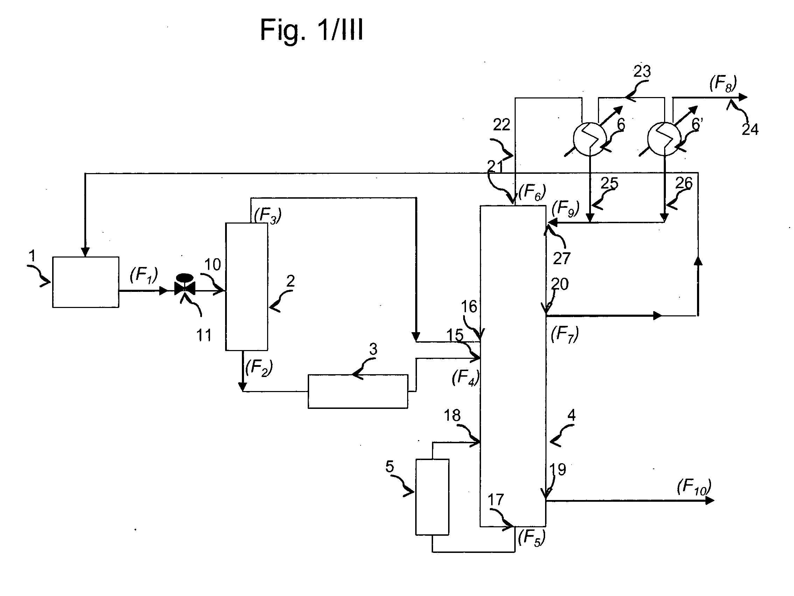

Un mode de réalisation pratique de l'invention est illustré par le dessin annexé sous forme de la figure 1

La figure 1 est une vue latérale schématique d'un appareillage adapté à la mise en oeuvre de l'invention.

L'appareillage utilisé est constitué de trois ensembles.

Le premier ensemble comprend le réacteur (1) équipé des moyens d'introduction (non représentés) du composé de type amine, du solvant et du phosgène éventuellement en mélange avec le solvant.

A la sortie du réacteur, on obtient un mélange réactionnel *(F₁)* comprenant essentiellement les chlorures de carbamyle, le solvant organique, le phosgène en excès, l'acide chlorhydrique formé et des impuretés formées au cours de la réaction.

Le mélange réactionnel *(F₁)* est convoyé entre le réacteur (1) et l'enceinte de séparation (2) par l'intermédiaire d'une tuyauterie qui est obligatoirement équipé d'une vanne de détente (11) lorsque la pression de la réaction est supérieure à la pression de distillation.

Le deuxième ensemble comprend une enceinte de séparation (2) et un préchauffeur (3).

Le flux *(F₁)* est introduit au niveau de (10) dans une enceinte de séparation (2) et l'on récupère en tête d'enceinte, un flux gazeux *(F₃)* comprenant de l'acide chlorhydrique et du phosgène et en pied d'enceinte, un flux liquide *(F₂)* comprenant essentiellement des chlorures de carbamyle, le solvant organique, du phosgène, de l'acide chlorhydrique résiduel et des impuretés.

Le flux liquide *(F₂)* traverse un préchauffeur (3) et en sortie, le flux réchauffé *(F₄)* est introduit en (15) dans une colonne de distillation (4).

Le flux gazeux *(F₃)* obtenu en tête de l'enceinte de séparation (2) est envoyé également dans la colonne de distillation, en (16) dans une zone au-dessus de (15) qui est l'alimentation de *(F₄).*

Le troisième ensemble comprend la colonne de distillation (4), un bouilleur (5) et un ou des condenseurs (6) et (6') placés en tête de colonne.

Le flux réchauffé *(F₄)* est introduit en (15), en dessous de (16) et subit une distillation réactive dans la colonne (4).

En pied de colonne en (17), le flux *(F₅)* est chauffé extérieurement à la colonne en faisant circuler le flux *(F₅)* dans une boucle qui traverse le bouilleur (5).

In est ensuite réintroduit latéralement en (18) sur la colonne de distillation.

Au niveau de la partie basse de la colonne, on récupère en (19), un flux *(F₁₀)* comprenant l'isocyanate attendu et des impuretés dans le solvant organique et du phosgène résiduel.

Dans une zone supérieure à l'alimentation du mélange réactionnel *(F₄)*, on soutire en (20), un flux *(F₇)* liquide comprenant majoritairement du phosgène qui peut éventuellement être recyclé à l'étape de phosgénation de l'amine.

En tête de distillation, on obtient une phase gazeuse *(F₆)* comprenant le phosgène et l'acide chlorhydrique.

En partie haute de la colonne, se trouvent les moyens pour traiter le flux gazeux issu de la colonne de distillation (4).

Le flux gazeux *(F₆)* comprenant du phosgène et de l'acide chlorhydrique, est récupéré en tête de colonne en (21)

L'enrichissement en acide chlorhydrique gazeux du flux *(F₆)* qui sort par la tuyauterie (22) se fait par condensation dans un premier condenseur (6) puis éventuellement dans un deuxième condenseur (6') relié à la sortie gazeuse du premier condenseur par la tuyauterie (23).

Le gaz chlorhydrique concentré *(F₈)* sort en (24).

Les liquides condensés en (25) et (26) comprenant essentiellement du phosgène et minoritairement de l'acide chlorhydrique sont réunis *(F₉)* et introduits latéralement en (27) en tête de colonne.

Dans ce mode de réalisation représenté par la figure 1, l'Homme du Métier peut si nécessaire ajuster les paramètres de contrôle à l'aide de vannes de régulation.

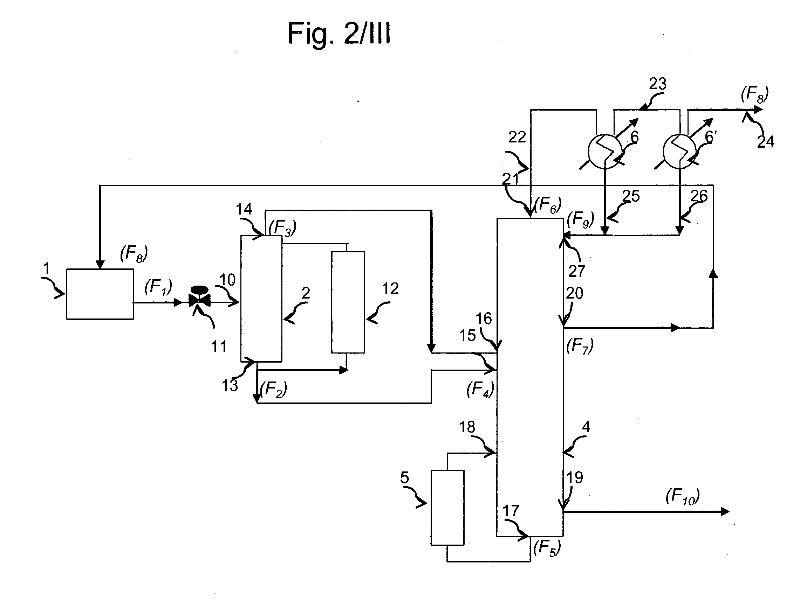

La figure 2 est une vue latérale schématique d'un appareillage identique à la figure 1 à l'exception du préchauffeur (3) qui est remplacé par un système de chauffage par circulation en boucle autour de l'enceinte 2, à travers un échangeur thermique (12).

La figure 3 est une vue latérale schématique d'un appareillage identique à la figure 1 mais inclut également une unité de déphosgénation du flux *(F₁₀)* récupéré en pied de la colonne de distillation (4) comprenant l'isocyanate attendu et des impuretés dans le solvant organique et du phosgène résiduel.

Le quatrième ensemble comprend les moyens pour traiter le flux liquide *(F₁₀)* issu en (19) c'est-à-dire une colonne de distillation (7), un bouilleur (8) et un ou deux condenseurs (9) et (9') placés en tête de colonne.

Le flux *(F₁₀)* est introduit en (28) dans une colonne à distillation (7) conçue pour récupérer en pied de colonne, en (29), le flux *(F₁₅)* d'isocyanate et des impuretés dans le solvant organique et en tête de colonne, en (30) le flux gazeux *(F₁₂)* comprenant essentiellement du phosgène, une fraction de solvant organique et de l'acide chlorhydrique résiduel.

En pied de colonne en (31), le flux *(F₁₁)* est chauffé extérieurement à la colonne en le faisant circuler dans une boucle qui traverse le bouilleur (8).

Il est ensuite réintroduit latéralement en (32) sur la colonne de distillation (7).

Au niveau de la partie basse de la colonne, on récupère en (29), un flux *(F₁₅)* tel que précédemment défini.

Le flux gazeux *(F₁₂)* est récupéré en tête de colonne en (30).

La récupération du phosgène à partir du flux *(F₁₂)* qui sort par la tuyauterie (33) se fait par condensation dans un premier condenseur (9) puis éventuellement dans un deuxième condenseur (9') relié à la sortie gazeuse du premier condenseur par la tuyauterie (34).

Les liquides condensés en (36) et (37) comprenant essentiellement du phosgène sont réunis en un flux dont une partie *(F₁₄)* alimente le reflux de la colonne (7) et l'autre partie *(F₁₃)* sortant en (35) qui peut être recyclé à l'étape de la phosgénation.

Le procédé de l'invention est particulièrement intéressant car il permet d'augmenter la productivité de la colonne de distillation réactive de 40 à 50 % en s'affranchissant des instabilités hydrodynamiques.

Le procédé de l'invention présente l'avantage de diminuer l'encrassement des bouilleurs si bien que le nombre d'arrêts pour nettoyage est diminué d'un tiers et le taux opératoire augmenté.

Le procédé de l'invention est particulièrement intéressant car il permet de rendre indépendantes l'optimisation, notamment par la température de réaction, du rendement en précurseurs d'isocyanate et l'optimisation du fonctionnement de la colonne de distillation réactive par la température de son alimentation, l'enjeu étant de 2 à 3 % de rendement.

Le procédé de l'invention présente l'avantage d'obtenir une meilleure efficacité énergétique de cette colonne.

Le procédé de l'invention présente l'avantage de diminuer la quantité d'acide chlorhydrique recyclée à la phosgénation par le phosgène issu de la tête de la deuxième colonne de distillation.

On donne ci-après, un exemple de réalisation pratique de l'invention donné à titre illustratif et sans caractère limitatif.

### Exemple

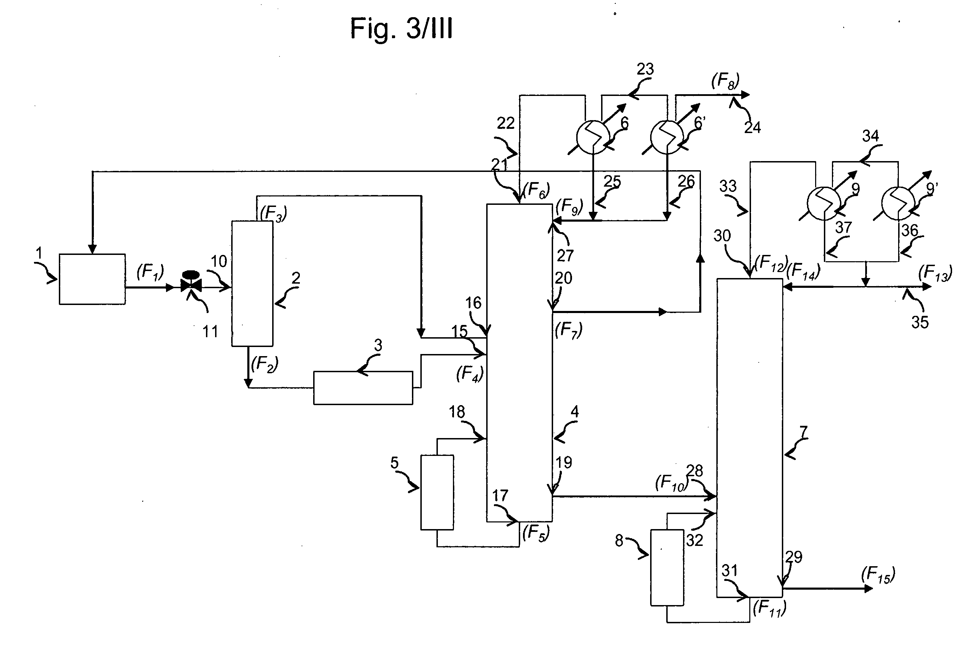

Le procédé décrit ci-après est mis en oeuvre dans un appareillage tel que schématisé par la figure 3.

Le réacteur (1), d'une capacité de 5 litres, est alimenté en continu par 485 kg/h de solution à 22 % de toluènediamine dans du monochlorobenzène et par 1007 kg/h de solution à 78 % de phosgène dans le monochlorobenzène.

La température du réacteur est de 135 °C et la pression de 45 bars.

L'enceinte de séparation (2) est à 16,4 bars, la température en pied est de 118 °C.

Le flux gazeux *(F₃)* séparé dans (2) est introduit en 16 dans la colonne de distillation réactive (4).

Le flux liquide séparé *(F₂)* est introduit dans l'échangeur de préchauffage (3).

A la sortie de l'échangeur (3), la température est de 140°C.

Le flux sortant de (3) est introduit en 15 dans la colonne de distillation réactive (4) qui opère sous une pression de 16,4 bars et une température en pied de 173°C.

Le flux *(F₁₀)* issu du pied de cette colonne en (19) est de 1097 kg/h contenant dans le monochlorobenzène, 12,8 % de toluènediisocyanate et 30 % de phosgène et 1,4 % d'impuretés.

Dans ledit flux, on ne constate pas la présence de chlorure de carbamyle.

On effectue ensuite la déphosgénation de *(F₁₀)* par distillation dans la colonne (7) à 185°C en pied et sous une pression de 2 bars.

On récupère en tête, le phosgène et un peu de solvant environ 10 % en poids par rapport au phosgène.

En (29), on obtient un flux *(F₁₅)* de toluènediisocyanate dans le monochlorobenzène à raison de 22 %. On élimine ensuite le solvant.

## Revendications

1. Procédé de préparation d'un composé de type isocyanate à partir de l'amine correspondante et de phosgène comprenant une étape de réaction sous pression, de l'amine et du phosgène, en présence d'un solvant organique suivie par une étape de distillation réactive sous pression **caractérisé par le fait qu'**il comprend à la sortie de l'opération de phosgénation, les étapes suivantes :
- une étape de séparation du mélange réactionnel (F₁) issu de l'étape de phosgénation en un flux gazeux d'acide chlorhydrique et de phosgène (F₃) et en un flux liquide (F₂) comprenant essentiellement les précurseurs d'isocyanate dans le solvant organique,
- une introduction du flux gazeux séparé (F₃) dans la colonne de distillation, de préférence à mi-hauteur,
- une étape de préchauffage du flux liquide (F₂) à une température comprise entre 100°C et 250°C,
- une introduction du flux préchauffé (F₄) dans la colonne de distillation, en dessous de l'alimentation du flux (F₃),
- une opération de distillation du flux (F₄) conçue pour obtenir :
□ en pied de distillation, (F₁₀), l'isocyanate attendu dans le solvant organique et du phosgène résiduel,
□ en tête de distillation, une phase gazeuse (F₆) comprenant du phosgène et l'acide chlorhydrique,
□ dans une zone supérieure à l'alimentation du mélange réactionnel (F₄), un flux liquide (F₇) comprenant très majoritairement du phosgène.

2. Procédé selon la revendication 1 **caractérisé par le fait que** le mélange réactionnel (F₁) est obtenu suite à la réaction de l'amine et du phosgène, en présence d'un solvant organique, à une température comprise entre 80°C et 250°C, sous une pression supérieure à la pression atmosphérique, de préférence comprise entre 5 et 80 bars.

3. Procédé selon l'une des revendications 1 et 2 **caractérisé par le fait que** l'amine répond à la formule suivante :
H₂N - R - NH₂ (I)
dans ladite formule :
- R représente un groupe divalent choisi parmi les groupes alkylène, cycloalkylène ou arylène ou un enchaînement desdits groupes,
de préférence l'amine est choisie parmi les diamines suivantes :
- l'hexaméthylènediamine,
- l'isophoronediamine,
- la 2,4-toluènediamine ou la 2,6-toluènediamine ou leur mélange, (TDA)
- la 4,4'- méthylènediphénylamine ou la 2,2'- méthylènediphénylamine ou la 2,4'-méthylènediphénylamine ou leur mélange (MDA),
- la 4,4'- méthylènedicyclohexyldiamine ou la 2,2'- méthylène- dicyclohexyldiamine ou la 2,4'- méthylènedicyclohexyldiamine ou leur mélange (H₁₂MDA).

4. Procédé selon la revendication 1 **caractérisé par le fait que** le solvant organique est un hydrocarbure aliphatique, cycloaliphatique ou aromatique halogène ou non ; un ester d'acide carboxylique aliphatique ou aromatique, de préférence le toluène, le monochorobenzène ou les dichlorobenzènes.

5. Procédé selon l'une des revendications 2 à 4 **caractérisé par le fait que** le rapport entre le nombre de moles de phosgène et le nombre de fonctions amino varie entre 3 et 6, de préférence entre 3,5 et 5,5.

6. Procédé selon l'une des revendications 1 à 5 **caractérisé par le fait que** l'on effectue le préchauffage du flux liquide (F₂) par passage dans un échangeur thermique, de préférence un échangeur tubes calandre chauffé par condensation de vapeur d'eau ou par un fluide caloporteur approprié, de préférence le solvant organique de la réaction, les esters lourds d'acides carboxylique, les éthers aromatiques plus préférentiellement l'oxyde de biphényle et/ou l'oxyde de benzyle, le diphényle, les terphényles, les autres polyphényles éventuellement partiellement hydrogénés, les huiles paraffiniques et/ou naphténiques, résidus de distillation du pétrole.

7. Procédé selon l'une des revendications 1 à 6 **caractérisé par le fait que** l'on effectue le préchauffage du flux liquide (F₂) en établissant une boucle de recirculation sur l'enceinte de séparation qui est chauffée par un échangeur thermique, notamment un échangeur tubes calandre : le flux recirculé sortant en pied d'enceinte, traversant de bas en haut un échangeur thermique est introduit, en sortie d'échangeur, en tête de l'enceinte de séparation.

8. Procédé selon l'une des revendications 1 à 7 **caractérisé par le fait que** l'opération de distillation du flux (F₄) est conduite sous une pression comprise entre 3 et 30 bars, de préférence ente 10 et 30 bars.

9. Procédé selon l'une des revendications 1 à 8 **caractérisé par le fait que** l'étape de réaction de phosgénation de l'amine est conduite à une pression supérieure à la pression de distillation réactive et **par le fait que** le mélange réactionnel (F₁), à la sortie de l'étape de réaction est soumis à une étape supplémentaire de détente de telle sorte que sa pression soit amenée au niveau de la pression de distillation, ladite détente étant de préférence assurée à l'aide d'une vanne de détente placée sur le conduit véhiculant le mélange réactionnel (F₁) de la zone de réaction à l'enceinte de séparation.

10. Procédé selon la revendication 9 **caractérisé par le fait que** la pression après détente est comprise entre 3 et 30 bars, de préférence ente 10 et 30 bars.

11. Procédé selon la revendication 1 **caractérisé par le fait que** le flux gazeux (F₆), en tête de colonne, comprenant majoritairement du phosgène et de l'acide chlorhydrique, est condensé de manière à récupérer un flux d'HCl gazeux (F₈) et un flux liquide (F₉) comprenant essentiellement du phosgène et minoritairement de l'acide chlorhydrique, ce dernier flux étant introduit, latéralement en tête de colonne, pour assurer le reflux dans la colonne.

12. Procédé selon l'une des revendications 1 à 10 **caractérisé par le fait que** l'on soumet le flux (F₁₀) recueilli en pied de distillation à une opération de déphosgénation, notamment en soumettant le flux (F₁₀) à une opération de distillation conçue pour obtenir :
- en pied de distillation, (F₁₅), l'isocyanate attendu dans le solvant organique et du phosgène résiduel,
- en tête de distillation, une phase gazeuse (F₁₂) comprenant du phosgène et une fraction de solvant organique.

13. Procédé selon la revendication 12 **caractérisé par le fait que** le flux gazeux (F₁₂), en tête de colonne comprenant du phosgène et une fraction de solvant organique est condensé de manière à récupérer un flux dont une partie (F₁₄) alimente le reflux de la colonne de distillation et l'autre partie (F₁₃) peut être recyclée à l'étape de la phosgénation.

14. Procédé selon l'une des revendications 1 à 13 **caractérisé par le fait que** l'on récupère l'isocyanate attendu à partir du flux (F₁₅), de préférence par distillation.

15. Installation permettant la mise en oeuvre du procédé décrit dans l'une des revendications 1 à 14 comprenant :
- un réacteur équipé de dispositifs d'introduction des réactifs,
- une enceinte de séparation assurant la détente éventuelle et la séparation du mélange réactionnel et des moyens de chauffage permettant d'augmenter la température du mélange réactionnel introduit dans la colonne de distillation,
- entre le réacteur et l'enceinte de séparation, éventuellement des moyens de détente, de préférence une vanne de réglage, permettant d'amener la pression de la réaction à une pression voisine de la pression de la distillation
- une première colonne de distillation équipée en pied de moyens de chauffage conçue pour séparer l'acide chlorhydrique et partiellement le phosgène du mélange réactionnel et pour dissocier les chlorures de carbamyle en isocyanate à partir dudit mélange,
- un ou deux condenseurs situés à la sortie du flux gazeux récupéré en tête de colonne de distillation conçus pour assurer le reflux dans la colonne de distillation,
- éventuellement une deuxième colonne de distillation dont l'entrée est alimentée par le pied de la première colonne, cette colonne étant conçue pour obtenir en pied, l'isocyanate attendu dans le solvant organique et les impuretés de réaction dans le solvant organique et en tête de colonne, le phosgène et une fraction de solvant,
- éventuellement un ou deux condenseurs situés à la sortie du flux gazeux récupéré en tête de deuxième colonne de distillation conçus pour assurer le reflux dans la colonne de distillation.

## Patentansprüche

1. Verfahren zur Herstellung einer Isocyanat-Zusammensetzung ausgehend von dem korrespondierenden Amin und Phosgen, umfassend einen Reaktionsschritt von Amin und Phosgen in Anwesenheit eines organischen Lösungsmittels unter Druck, gefolgt von einem Destillationsreaktionsschritt unter Druck, **dadurch gekennzeichnet, dass** das Verfahren nach Beendigung der Phosgenierung die folgenden Schritte umfasst:
- einen Auftrennungsschritt der reaktiven Mischung (F₁), die aus dem Phosgenierungsschritt resultiert, in einen gasförmigen Strom Salzsäure und Phosgen (F₃) und in einen flüssigen Strom (F₂), im Wesentlichen umfassend Isocyanat-Vorstufen im organischen Lösungsmittel,
- Einführung des abgetrennten gasförmigen Stroms (F₃) in die Destillationskolonne, vorzugsweise auf halber Höhe,
- einen Vorheizschritt des flüssigen Stroms (F₂) auf eine Temperatur zwischen 100 °C und 250 °C,
- Einführung des vorgeheizten Stroms (F₄) in die Destillationskolonne, unterhalb der Einführungsstelle des Stroms (F₃),
- einen Destillationsvorgang des Stroms (F₄) konzipiert zum Erhalt von:
o Isocyanat, welches im organischen Lösungsmittel verbleibt, und restliches Phosgen am Fuß der Destillation, (F₁₀),
∘ einer gasförmigen Phase, umfassend Phosgen und Salzsäure, an der Spitze der Destillation,
o einem flüssigen Strom (F₇), umfassend hauptsächlich Phosgen, in einem Bereich oberhalb der Einführungsstelle der reaktiven Mischung (F₄).

2. Verfahren gemäß Anspruch 1, **dadurch gekennzeichnet, dass** die reaktive Mischung *(F₁)* im Anschluss an die Reaktion des Amins und des Phosgens in Anwesenheit eines organischen Lösungsmittels bei einer Temperatur zwischen 80 °C und 250 °C unter einem Druck, welcher höher als der Atmosphärendruck ist, bevorzugt zwischen 5 und 80 bar, erhalten wird.

3. Verfahren gemäß einem der Ansprüche 1 und 2, **dadurch gekennzeichnet, dass** das Amin folgende Formel hat:
H₂N - R - NH₂ (I)
wobei in der Formel
- R eine divalente Gruppe ausgewählt aus der Gruppe bestehend aus Alkylen, Cycloalkylen oder Arylen oder einer Verkettung selbiger Gruppen ist,
wobei das Amin bevorzugt ausgewählt ist aus folgenden Diaminen:
- Hexamethylendiamin,
- Isophorondiamin,
- 2,4-Toluoldiamin oder 2,6-Toluoldiamin oder eine Mischung daraus (TDA),
- 4-4'-Methylendiphenylamin oder 2,2'-Methylendiphenylamin oder 2,4'-Methylendiphenylamin oder eine Mischung daraus (MDA),
- 4,4'-Methylendicyclohexyldiamin oder 2,2'-Methylendicyclohexyldiamin oder 2,4'-Methylendicyclohexyldiamin oder eine Mischung daraus (H₁₂MDA).

4. Verfahren gemäß Anspruch 1, **dadurch gekennzeichnet, dass** das organische Lösungsmittel ein aliphatischer, cycloaliphatischer oder aromatischer Kohlenwasserstoff mit oder ohne Halogen, ein aliphatischer oder aromatischer Carbonsäureester, bevorzugt Toluol, Monochlorbenzol oder ein Dichlorbenzol ist.

5. Verfahren gemäß einem der Ansprüche 2 bis 4, **dadurch gekennzeichnet, dass** das Verhältnis zwischen der Anzahl an Molen Phosgen und der Anzahl an Aminfunktionalitäten zwischen 3 und 6, bevorzugt zwischen 3,5 und 5,5 liegt.

6. Verfahren gemäß einem der Ansprüche 1 bis 5, **dadurch gekennzeichnet, dass** das Vorheizen des flüssigen Stroms (F₂) mittels Durchleiten durch einen Wärmetauscher, bevorzugt eines beheizten, zylindrischen tubulären Wärmetauschers durch Kondensation von Wasserdampf oder durch eine geeignete wärmeleitende Flüssigkeit, bevorzugt das organische Lösungsmittel der Reaktion, schwere Carbonsäureester, aromatische Ether, am meisten bevorzugt das Oxid des Biphenyls und/oder das Oxid des Benzyls, Diphenyls, Triphenyls, andere Polyphenyle, unter Umständen teilweise hydriert, Paraffinöle und/oder Naphthenöle, oder Rückstände der Destillation von Petroleum erreicht wird.

7. Das Verfahren gemäß einem der Ansprüche 1 bis 6, **dadurch gekennzeichnet, dass** das Vorheizen des flüssigen Stroms (F₂) über eine Rücklaufschlaufe über dem Auftrennungsbereich, die durch einen Wärmetauscher beheizt wird, insbesondere einen zylindrischen, tubulären Wärmetauscher, erreicht wird, wobei der rückgeleitete Strom, der am Fuß dieses Bereiches austritt und der von unten nach oben den Wärmetauscher durchläuft, nach Verlassen des Wärmetauschers, an der Spitze des Auftrennungsbereiches eingeleitet wird.

8. Verfahren gemäß einem der Ansprüche 1 bis 7, **dadurch gekennzeichnet, dass** die Destillation des Stroms (F₄) unter Druck zwischen 3 und 30 bar, bevorzugt zwischen 10 und 30 bar durchgeführt wird.

9. Verfahren gemäß einem der Ansprüche 1 bis 8, **dadurch gekennzeichnet, dass** der Reaktionsschritt der Phosgenierung des Amins bei einem Druck, welcher höher ist als der der reaktiven Destillation, durchgeführt wird, und dass die reaktive Mischung (F₁), nach Beendigung des Reaktionsschrittes einem zusätzlichen Abzugsschritt unterworfen wird, derart, dass der Druck auf das Druckniveau der Destillation herangeführt wird, wobei der Abzug bevorzugt durch ein Abzugsventil, welches über der Leitung, welche die reaktive Mischung (F₁) vom Reaktionsbereich zum Auftrennungsbereich transportiert platziert ist, gewährleistet wird.

10. Verfahren gemäß Anspruch 9, **dadurch gekennzeichnet, dass** der Druck nach Abzug zwischen 3 und 30 bar, bevorzugt zwischen 10 und 30 bar liegt.

11. Verfahren gemäß Anspruch 1, **dadurch gekennzeichnet, dass** der gasförmige Strom an der Spitze der Kolonne, welcher hauptsächlich Phosgen und Salzsäure umfasst, auf eine Art und Weise kondensiert wird, dass ein gasförmiger HCl-Strom (F₈) und ein flüssiger Strom (F₉), im Wesentlichen umfassend Phosgen und Restmengen an Salzsäure, erhalten wird, wobei letztgenannter Strom seitlich an der Spitze der Kolonne eingeleitet wird, um Rückfluss innerhalb der Kolonne zu gewährleisten.

12. Verfahren gemäß einem der Ansprüche 1 bis 10, **dadurch gekennzeichnet, dass** der Strom (F₁₀), der am Fuß der Destillation aufgefangen wurde, einer Dephosgenierung unterworfen wird, insbesondere indem der Strom (F₁₀) einer Destillation unterworfen wird, die dafür ausgelegt ist, Folgendes zu erhalten:
- Isocyanat, welches im organischen Lösungsmittel verbleibt, und restliches Phosgen am Fuß der Destillation, (F₁₀),
- eine gasförmige Phase (F₁₂), umfassend Phosgen und eine Fraktion des organischen Lösungsmittels, an der Spitze der Destillation.

13. Verfahren gemäß Anspruch 12, **dadurch gekennzeichnet, dass** der gasförmige Strom (F₁₂) am Kopf der Kolonne, welcher Phosgen und eine Fraktion des organischen Lösungsmittels umfasst, auf eine Art und Weise kondensiert wird, dass ein Strom zurückgewonnen wird, dessen einer Teil (F₁₄) den Rückfluss innerhalb der Destillationskolonne versorgt und dessen anderer Teil (F₁₃) im Phosgenierungsschritt wiederverwendet werden kann.

14. Verfahren gemäß einem der Ansprüche 1 bis 13, **dadurch gekennzeichnet, dass** das Isocyanat, welches ab Strom (F₁₅) erwartet wird, zurückgewonnen wird, bevorzugt mittels Destillation.

15. Anlage, die die Durchführung des Verfahrens gemäß einem der Ansprüche 1 bis 14 ermöglicht, umfassend:
- Einen Reaktor, welcher ausgestattet ist mit einer Vorrichtung zur Einleitung der Reaktanden,
- Einen Bereich zur Auftrennung, welcher den etwaigen Abzug und die Trennung der reaktiven Mischung gewährleistet, und Heizvorrichtungen, welche die Erhöhung der Temperatur der reaktiven Mischung, welche in die Destillationskolonne eingeleitet ist, ermöglichen.
- gegebenenfalls Abzugsvorrichtungen zwischen dem Reaktor und dem Auftrennungsbereich, bevorzugt ein regelbares Ventil, welches das Heranführen der des Reaktionsdrucks nahe an den Destillationsdruck ermöglicht,
- eine erste Destillationskolonne, welche am Fuß mit Heizvorrichtungen ausgestattet ist, und die zur Abtrennung der Salzsäure und einem Teil des Phosgens von der reaktiven Mischung und zum Absondern der Carbamylchloride im Isocyanat von der Mischung konzipiert ist,
- einen oder zwei Kondensoren, welche sich am Austrittspunkt des zurückgewonnenen gasförmigen Stroms am Kopf der Destillationskolonne befinden und welche konzipiert sind, den Rückfluss innerhalb der Kolonne zu gewährleisten,
- gegebenenfalls eine zweite Destillationskolonne, deren Einleitung durch den Fuß der ersten Kolonne gespeist wird, wobei diese Kolonne dazu konzipiert ist, das Isocyanat, welches im organischen Lösungsmittel verbleibt, sowie Verunreinigungen der Reaktion im organischen Lösungsmittel am Fuß, und Phosgen und eine Fraktion des Lösungsmittels an der Spitze der Kolonne zu erhalten,
- gegebenenfalls einen oder zwei Kondensoren, welche sich am Austrittspunkt des zurückgewonnenen, gasförmigen Stroms an der Spitze der zweiten Destillationskolonne befinden und welche konzipiert sind, den Rückfluss innerhalb der Kolonne zu gewährleisten.

## Claims

1. Process for the preparation of a compound of the isocyanate type from the corresponding amine and phosgene, comprising a step of reacting, under pressure, the amine and phosgene in the presence of an organic solvent, followed by a step of reactive distillation under pressure, **characterised in that** it comprises, at the end of the phosgenation operation, the following steps:
- a step of separating the reaction mixture (F₁) obtained in the phosgenation step into a gaseous stream of hydrochloric acid and phosgene (F₃) and a liquid stream (F₂) comprising substantially the isocyanate precursors in the organic solvent,
- introducing the separated gaseous stream (F₃) into the distillation column, preferably at mid-height,
- a step of preheating the liquid stream (F₂) to a temperature of from 100°C to 250°C,
- introducing the preheated stream (F₄) into the distillation column below the feed of stream (F₃),
- an operation of distilling stream (F₄), which is designed to obtain:
□ at the bottom of the distillation column, (F₁₀), the expected isocyanate in the organic solvent, and residual phosgene,
□ at the top of the distillation column, a gaseous phase (F₆) comprising phosgene and hydrochloric acid,
□ in a zone above the feed of the reaction mixture (F₄), a liquid stream (F₇) comprising for the most part phosgene.

2. Process according to claim 1, **characterised in that** the reaction mixture (F₁) is obtained following the reaction of the amine and phosgene, in the presence of an organic solvent, at a temperature of from 80°C to 250°C, under a pressure greater than atmospheric pressure, preferably from 5 to 80 bar.

3. Process according to either claim 1 or claim 2, **characterised in that** the amine corresponds to the following formula:
H₂N - R - NH₂ (I)
wherein:
- R represents a divalent group chosen from the groups alkylene, cycloalkylene or arylene or a chain of said groups,
preferably the amine is chosen from the following diamines:
- hexamethylenediamine,
- isophoronediamine,
- 2,4-toluenediamine or 2,6-toluenediamine or a mixture thereof (TDA),
- 4,4'-methylenediphenylamine or 2,2'-methylenediphenylamine or 2,4'-methylenediphenylamine or a mixture thereof (MDA),
- 4,4'-methylenedicyclohexyldiamine or 2,2'-methylenedicyclohexyldiamine or 2,4'-methylenedicyclohexyldiamine or a mixture thereof (H₁₂MDA).

4. Process according to claim 1, **characterised in that** the organic solvent is a halogenated or non-halogenated aliphatic, cycloaliphatic or aromatic hydrocarbon; an aliphatic or aromatic carboxylic acid ester, preferably toluene, monochlorobenzene or dichlorobenzenes.

5. Process according to any one of claims 2 to 4, **characterised in that** the ratio between the number of moles of phosgene and the number of amino functions varies from 3 to 6, preferably from 3.5 to 5.5.

6. Process according to any one of claims 1 to 5, **characterised in that** the preheating of the liquid stream (F₂) is carried out by passing it through a heat exchanger, preferably a shell and tubes exchanger heated by condensation of steam or by a suitable heat transfer fluid, preferably the organic solvent of the reaction, heavy esters of carboxylic acids, aromatic ethers, more preferably biphenyl oxide and/or benzyl oxide, diphenyl, terphenyls, other optionally partially hydrogenated polyphenyls, paraffin and/or naphthenic oils, petroleum distillation residues.

7. Process according to any one of claims 1 to 6, **characterised in that** the preheating of the liquid stream (F₂) is carried out by establishing on the separating vessel a recirculation loop which is heated by a heat exchanger, especially a shell and tubes exchanger: the recirculated stream leaving at the bottom of the vessel, passing from bottom to top through a heat exchanger is introduced, at the outlet of the exchanger, at the top of the separating vessel.

8. Process according to any one of claims 1 to 7, **characterised in that** the operation of distilling the stream (F₄) is performed under a pressure of from 3 to 30 bar, preferably from 10 to 30 bar.

9. Process according to any one of claims 1 to 8, **characterised in that** the step of phosgenation reaction of the amine is performed at a pressure greater than the reactive distillation pressure, and **in that** the reaction mixture (F₁), at the end of the reaction step, is subjected to an additional expansion step so that its pressure is brought to the level of the distillation pressure, said expansion preferably being carried out with the aid of an expansion valve located on the pipe carrying the reaction mixture (F₁) from the reaction zone to the separating vessel.

10. Process according to claim 9, **characterised in that** the pressure after expansion is from 3 to 30 bar, preferably from 10 to 30 bar.

11. Process according to claim 1, **characterised in that** the gaseous stream (F₆), at the top of the column, comprising for the most part phosgene and hydrochloric acid, is condensed so as to recover a gaseous stream of HCl (F₈) and a liquid stream (F₉) comprising substantially phosgene and a minor amount of hydrochloric acid, the latter stream being introduced, laterally at the top of the column, in order to effect reflux in the column.

12. Process according to any one of claims 1 to 10, **characterised in that** the stream (F₁₀) collected at the bottom of the distillation column is subjected to a dephosgenation operation, especially by subjecting the stream (F₁₀) to a distillation operation designed to obtain:
- at the bottom of the distillation column, (F₁₅), the expected isocyanate in the organic solvent, and residual phosgene,
- at the top of the distillation column, a gaseous phase (F₁₂) comprising phosgene and an organic solvent fraction.

13. Process according to claim 12, **characterised in that** the gaseous stream (F₁₂), at the top of the column, comprising phosgene and an organic solvent fraction is condensed so as to recover a stream of which one part (F₁₄) feeds the reflux of the distillation column and the other part (F₁₃) can be recycled to the phosgenation step.

14. Process according to any one of claims 1 to 13, **characterised in that** the expected isocyanate is recovered from stream (F₁₅), preferably by distillation.

15. Installation with which the process described in any one of claims 1 to 14 can be carried out, comprising:
- a reactor equipped with devices for introducing the reagents,
- a separating vessel which effects expansion, where appropriate, and separation of the reaction mixture, and heating means allowing the temperature of the reaction mixture introduced into the distillation column to be increased,
- between the reactor and the separating vessel, optionally expansion means, preferably a regulating valve, allowing the reaction pressure to be brought to a pressure close to the distillation pressure,
- a first distillation column equipped at the bottom with heating means designed to separate the hydrochloric acid and part of the phosgene from the reaction mixture and to decompose the carbamyl chlorides into isocyanate starting from said mixture,
- one or two condensers which are situated at the outlet of the gaseous stream recovered at the top of the distillation column and which are designed to effect reflux in the distillation column,
- optionally a second distillation column, the inlet of which is fed by the bottom of the first column, this column being designed to obtain, at the bottom, the expected isocyanate in the organic solvent and the reaction impurities in the organic solvent and, at the top of the column, phosgene and a solvent fraction,
- optionally one or two condensers which are situated at the outlet of the gaseous stream recovered at the top of the second distillation column and which are designed to effect reflux in the distillation column
